# EUROPEAN PATENT APPLICATION

(11) **EP 4 830 722 A1**
(43) Date of publication of application: **28.10.2026**
(21) Application number: 24894509.9
(22) Date of filing: 14.11.2024
(51) Int. Cl.: A61K 41/00, A61K 47/69, A61K 9/51, A61K 49/22

(54) **METHOD FOR MANUFACTURING NANOBUBBLE-BASED DRUG CARRIER USING FOCUSED ULTRASOUND TECHNOLOGY AND NANOBUBBLE-BASED DRUG CARRIER MANUFACTURED THEREBY**

(30) Priority: 23.11.2023 KR 20230164585; 01.11.2024 KR 20240153263
(71) Applicant: Fust Lab. Co., Ltd., Yuseong-gu Daejeon 34015 (KR)
(72) Inventor: HWANGBO, Minsung, Daejeon 34127 (KR); LEE, Eun Bi, Daejeon 34007 (KR)
(74) Representative: RGTH
(86) International application number: PCT/KR2024/017950
(87) International publication number: WO 2025/110616

(57) **Abstract**

The present disclosure relates to a method for manufacturing a nanobubble-based drug delivery vehicle using a focused ultrasonic technology, and a nanobubble-based drug delivery vehicle manufactured thereby, and more specifically, to a method for manufacturing a drug delivery vehicle using a focused ultrasonic technology, the drug delivery vehicle including a shell, and a drug and nanobubbles contained inside the shell, and a nanobubble-based drug delivery vehicle manufactured thereby.

## Description

### [Technical Field]

The present disclosure relates to a method for manufacturing a nanobubble-based drug delivery vehicle using a focused ultrasonic technology, and a nanobubble-based drug delivery vehicle manufactured thereby, and more specifically, to a method for manufacturing a drug delivery vehicle using a focused ultrasonic technology, the drug delivery vehicle including a shell, and a drug and nanobubbles contained inside the shell, and a nanobubble-based drug delivery vehicle manufactured thereby.

### [Background Art]

Drug delivery systems are one of the key technologies in the field of nanomedicines. Drug delivery technologies based on nanomaterials have shown a steady increase since 1997. Active technology development has been observed in the order of China, Korea, the United States, Japan, and Europe, based on the patent applications primarily filed by research institutes such as universities. In recent years, active development has been progressing in various fields, including lipid nanoparticle (LNP) technology, nanoemulsion technology, and nanoliposomes.

In particular, as the field of nanomedicines with high absorption rates in the human body is attracting attention, there is a continuous demand for technologies for delivering a specific amount of nano-sized drugs to lesion sites. In this regard, although technologies for manufacturing microbubbles currently exist, there are problems in that microbubbles have a low absorption rate in the human body due to their relatively large size, and in that the microbubbles manufactured with current technologies are not uniform in size, resulting in inconsistencies in the amount of drug delivered through the bubbles. Furthermore, there is no technology for delivering nano-sized drugs to areas surrounding a lesion, and thus such demands have not been resolved.

In addition, reducing agents, surfactants, and organic solvents used to control the size of substances used in current drug delivery systems are toxic, making it difficult to use them in objects to be applied in vivo as drug delivery vehicles. Additionally, even when surfactants and organic solvents are used, the size of the substances manufactured is not uniform, resulting in poor bioavailability. In addition, to overcome these drawbacks, drug delivery vehicles encapsulated using proteins and phospholipids with high reducing power have been proposed. However, their types and concentrations are limited, and their therapeutic efficacy is very low due to the protein corona phenomenon in which numerous proteins present in the human body adhere to the drug delivery vehicles. Additionally, side effects such as toxicity have been confirmed, as the drug delivery vehicles affect non-target organs.

Under such situations, there is a continued demand for a method for manufacturing a nanobubble-based drug delivery vehicle and for a nanobubble-based drug delivery vehicle, which enable selective treatment by guiding drugs to lesion sites and thereby minimize drug side effects, allow delivery of nano-sized drugs with uniform content, and maximize therapeutic efficacy due to a high absorption rate in the human body.

### [Disclosure]

### [Technical Problem]

An object of the present disclosure is to provide a method for manufacturing a drug delivery vehicle using focused ultrasound.

Another object of the present disclosure is to provide a method for manufacturing a drug delivery vehicle with maximized bioavailability by delivering a nano-sized drug with uniform content.

Still another object of the present disclosure is to provide a method for manufacturing a drug delivery vehicle that can maximize therapeutic effects and minimize drug-induced side effects by using a nano-sized drug with a high absorption rate in the human body .

Yet another object of the present disclosure is to provide a drug delivery vehicle including a shell, and a drug and a first nanobubble.

Still yet another object of the present disclosure is to provide a drug delivery vehicle manufactured by a focused ultrasonic device.

The technical problems to be solved by the present disclosure are not limited to the aforementioned problems, and any other technical problems to be solved by the present disclosure not mentioned herein will be clearly understood from the following description by a person having ordinary knowledge in the technical field to which the present disclosure belongs ("one skilled in the art").

### [Technical Solution]

In order to address the objects as described above, a method for manufacturing a drug delivery vehicle using focused ultrasound according to the present disclosure may be a method for manufacturing a drug delivery vehicle using focused ultrasound, the method including: manufacturing a first solution; manufacturing a second solution; and manufacturing a third solution by mixing the first solution and the second solution and irradiating the mixture with focused ultrasound, the third solution including a drug delivery vehicle including a shell, and a drug and first nanobubbles contained inside the shell.

For example, a uniformity of the drug delivery vehicle, represented by a poly-dispersity index (PDI), may be greater than 0 and equal to or less than 0.3.

For example, a size of the drug delivery vehicle may be determined based on at least one of size control factors including a focused ultrasound frequency, an ultrasound irradiation time, an ultrasound intensity, a phase transition temperature of a material, and a mixing speed of a solution.

For example, irradiation conditions of the focused ultrasound may be 10 to 100 W and 200 to 800 kHz.

For example, the first solution may be heated to a temperature ranging from about 25°C to about 80°C.

For example, the first solution may be heated to a temperature ranging from about 25°C to about 80°C.

For example, the second solution may be heated to a temperature ranging from about 50°C to about 110°C.

For example, the first solution may be heated to a temperature ranging from about 60°C to about 80°C.

For example, the first solution may be injected at a rate of 0.3 to 5.0 mL/min and mixed.

For example, the second solution may be injected at a rate of 10 to 100 mL/min.

For example, the drug delivery vehicle may have second nanobubbles formed on a surface of the shell.

For example, the drug delivery vehicle may have a decrease in absolute zeta potential as second nanobubbles are formed on a surface of the shell.

In addition, in order to address the objects as described above, a drug delivery vehicle according to the present disclosure may be a drug delivery vehicle including a shell; and a drug and first nanobubbles contained inside the shell, and having a uniformity within a predetermined range, wherein the uniformity of the drug delivery vehicle, represented by a poly-dispersity index (PDI), is greater than 0 and equal to or less than 0.3.

For example, the drug delivery vehicle may be one or more selected from the group consisting of lecithin, cholesterol, PEG-PCL (poly(ethylene glycol)-poly(ε-caprolactone)), DSPC (distearoylphosphatidylcholine), DODMA (1,2-dioleyloxy-3-(dimethylamino)propane, N,N-dimethyl-2,3-bis[(9Z)-9-octadecen-1-yloxy]-1-propanamine), PLGA (poly(lactic-co-glycolic acid)), PLA (polylactic acid), and PVA (polyvinyl alcohol).

### [Advantageous Effects]

According to the present disclosure, a method for manufacturing a nanobubble-based drug delivery vehicle with maximized bioavailability by delivering a nano-sized drug with uniform content, and a nanobubble-based drug delivery vehicle manufactured thereby can be provided.

According to the present disclosure, a method for manufacturing a nanobubble-based drug delivery vehicle that can maximize therapeutic effects and minimize drug-induced side effects by using a nano-sized drug with a high absorption rate in the human body, and a nanobubble-based drug delivery vehicle manufactured thereby can be provided.

According to the present disclosure, a method for manufacturing a nanobubble-based drug delivery vehicle that can maximize therapeutic effects and minimize drug-induced side effects by selectively delivering a drug to a lesion site using magnetic nanoparticles, and a nanobubble-based drug delivery vehicle manufactured thereby can be provided.

According to the present disclosure, a method for manufacturing a nanobubble-based drug delivery vehicle that enables imaging to identify a location of a therapeutic agent carried in a nanobubble by using light emitted from a bubble surface, and a nanobubble-based drug delivery vehicle manufactured thereby can be provided.

According to the present disclosure, a drug delivery vehicle having excellent functionality can be provided efficiently using a focused ultrasonic device.

The excellent and/or useful effects according to the present disclosure are not limited to the effects of the present disclosure described above, and one skilled in the art will also be able to obviously recognize other excellent and/or useful effects of the present disclosure, which are not explicitly disclosed in the present specification, based on the disclosure of the present specification, and it should be understood that these are intentionally disclosed by the present specification and are obviously included in the scope of the present disclosure.

### [Description of Drawings]

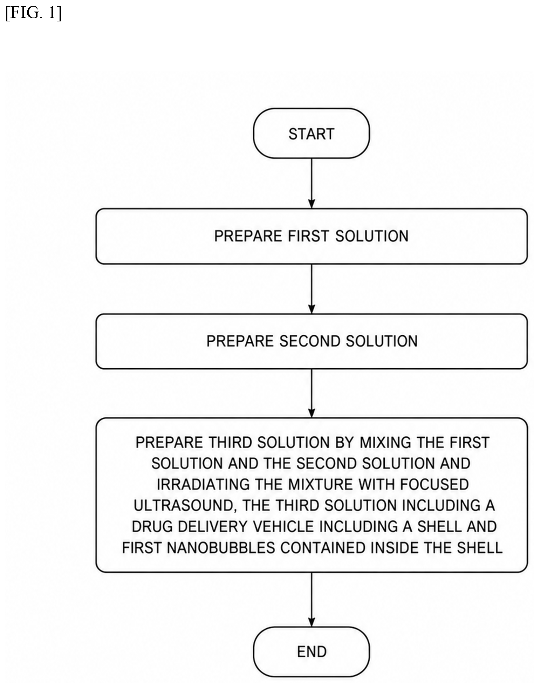

FIG. 1 is a diagram schematically showing a method for manufacturing a drug delivery vehicle using focused ultrasound according to an exemplary embodiment of the present disclosure.

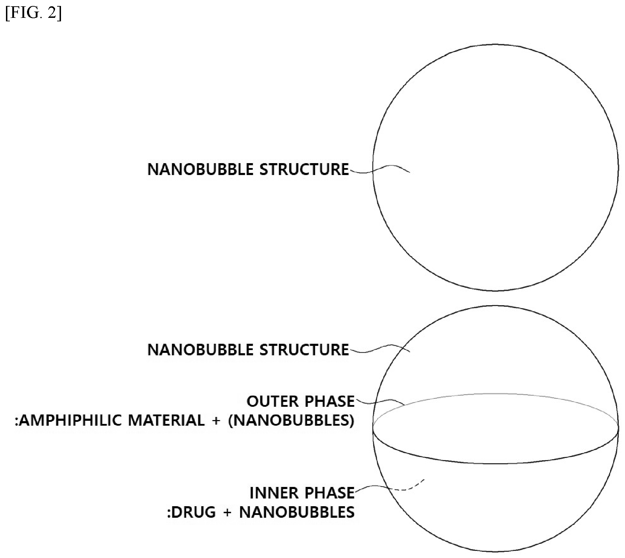

FIG. 2 is a diagram schematically showing a structure of a drug delivery vehicle according to an exemplary embodiment of the present disclosure.

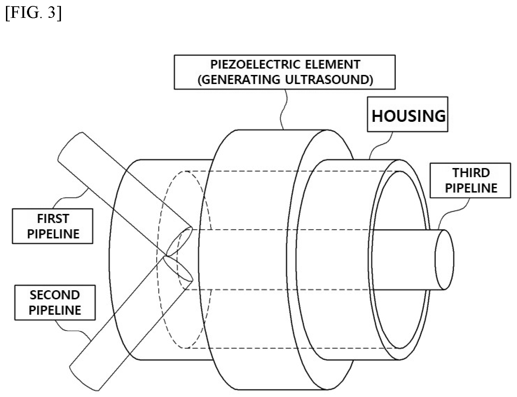

FIG. 3 is a diagram schematically showing a process for manufacturing a drug delivery vehicle according to an exemplary embodiment of the present disclosure using a focused ultrasonic device.
FIG. 4 is a diagram showing the results of visual observation of a drug delivery vehicle according to an exemplary embodiment of the present disclosure.
FIG. 5 is a diagram showing the results of particle size and size distribution measured using DLS for a drug delivery vehicle according to an exemplary embodiment of the present disclosure.
FIG. 6 is a diagram showing the results of Turbiscan for a drug delivery vehicle according to an exemplary embodiment of the present disclosure.
FIG. 7 is a diagram showing a cryo-EM image of liposomes included in a drug delivery vehicle according to an exemplary embodiment of the present disclosure.
FIG. 8 is a diagram showing a cryo-EM image of liposomes included in a drug delivery vehicle according to an exemplary embodiment of the present disclosure.

### [Best Mode]

Hereinafter, the present disclosure will be described in detail.

The terms or words used in the present specification and the claims are not intended to be construed as limited to their commonly used dictionary meanings, and a person having ordinary knowledge in the technical field to which the present disclosure belongs will clearly understand that such terms or words have been used in the sense intended to convey the meaning of the present disclosure within the scope of expressing the ideas that the present specification and the claims intend to obviously convey.

In addition, it will be clearly understood by one skilled in the art that the exemplary embodiments described in the present specification and the configurations described in the exemplary embodiments are merely preferred exemplary embodiments presented as examples to enable one skilled in the art to understand and reproduce the present disclosure, and are not intended to limit the present disclosure thereto.

In addition, the description and specific implementation of each configuration described in the present specification can be obviously applied to other respective descriptions and implementations. That is, it will be clearly understood by one skilled in the art that all combinations of the various configurations and specific implementations disclosed in the present specification fall within the scope of the present disclosure.

The term 'and/or' as used herein is a term that includes each of the mentioned items and all combinations of one or more thereof. In addition, singular terms include plurals unless otherwise stated.

The terms 'comprise (include)' and/or 'comprising (including)' used in the present specification are terms that do not exclude the presence or addition of items other than those mentioned.

The numerical range indicated by the term 'to' in the present specification indicates a numerical range that includes the values described before and after the term as the lower limit and the upper limit, respectively. When a plurality of numerical values are disclosed as the upper and lower limits of an arbitrary numerical range, the numerical range disclosed in the present specification can be understood as an arbitrary numerical range that uses any one of the plurality of lower limit values as the lower limit value and any one of the plurality of upper limit values as the upper limit value, respectively.

The terms "about" or "approximately" as used herein, when used, mean a value or numerical range within 10% of the value or numerical range stated after the term.

The terms or words used in the present specification and the claims should not be construed as limited to their ordinary dictionary meanings, but should be construed as meanings and concepts consistent with the technical spirit of the present invention, in accordance with the principle that an inventor can appropriately define the term in order to best describe the invention. Therefore, it should be understood that the configurations illustrated in the exemplary embodiments described in the present specification are only the most preferred exemplary embodiments of the present invention and do not represent all of the technical spirits of the present invention, and that there may be various equivalents and variations that can replace the foregoing at the time of filing of the present application.

Note that each of the descriptions and exemplary embodiments disclosed in the present specification can also be applied to other respective descriptions and implementations. That is, all combinations of the various elements disclosed in the present specification fall within the scope of the present invention, and descriptions omitted in one exemplary embodiment can be interpreted in the same manner as described in other exemplary embodiments. In addition, the scope of the present disclosure should not be construed as limited by the specific descriptions set forth below.

According to an aspect of the present disclosure, a method for manufacturing a drug delivery vehicle using focused ultrasound may be provided.

As an example, the method for manufacturing a drug delivery vehicle using focused ultrasound may be a method for manufacturing a drug delivery vehicle using focused ultrasound, the method including the steps of: manufacturing a first solution; manufacturing a second solution; and manufacturing a third solution by mixing the first solution and the second solution and irradiating the mixture with focused ultrasound, the third solution including a drug delivery vehicle including a shell, and a drug and first nanobubbles contained inside the shell.

As an example, the first solution may be a solution in which one or more materials selected from the group consisting of lecithin, cholesterol, PEG-PCL (poly(ethylene glycol)-poly(ε-caprolactone)), DSPC (distearoylphosphatidylcholine), DODMA (1,2-dioleyloxy-3-(dimethylamino)propane, N,N-dimethyl-2,3-bis[(9Z)-9-octadecen-1-yloxy]-1-propanamine), PLGA (poly(lactic-co-glycolic acid)), PLA (polylactic acid), and PVA (polyvinyl alcohol) are dissolved in a solvent.

As an example, the material of the first solution may be included in an amount of 0.5 to 1.0 part by weight based on 100 parts by weight of the entire first solution. If the material of the first solution is included in an amount of less than 0.5 part by weight based on 100 parts by weight of the entire first solution, there is a problem in that nanobubbles are not formed well. If the material of the first solution is included in an amount of more than 1.0 part by weight based on 100 parts by weight of the entire first solution, there is a problem in that sizes of the formed nanobubbles are not uniform and the nanobubbles are formed without internal cavities. Therefore, it is preferable that the content of the material of the first solution satisfies the above numerical range. Preferably, the material of the first solution may be included in an amount of 0.6 to 0.9 part by weight, more preferably 0.7 to 0.8 part by weight, and most preferably 0.75 part by weight based on 100 parts by weight of the entire first solution.

As an example, in the first step, the solvent may be an organic solvent, and the organic solvent may be an organic solvent including tetrahydrofuran (THF).

As an example, in the first step, a volume of the solvent may be 1.5 to 2.5 parts by volume based on 100 parts by volume of the entire first solution.

As an example, the second solution may be a solution in which a drug is dissolved in a solvent. For example, the solvent may be one or more selected from the group consisting of a lipophilic solvent and a hydrophilic solvent.

As an example, irradiation conditions of the focused ultrasound may be 10 to 100 W and 200 to 800 kHz.

As an example, the first nanobubbles of the third step may have an average diameter of 10 nm or more and 200 nm or less. When the average diameter of the first nanobubbles falls within the specific numerical range, the drug delivery vehicle can have excellent in vivo membrane permeability due to its small size. For example, the drug delivery vehicle may be designed to penetrate in vivo barriers with different permeabilities, such as cell membranes and the blood-brain barrier, by appropriately adjusting the average diameter of the first nanobubbles within the numerical range.

As an example, a uniformity of the drug delivery vehicle, represented by a poly-dispersity index (PDI), may be greater than 0 and equal to or less than 0.3.

As an example, a size of the drug delivery vehicle may be determined based on at least one of size control factors including a focused ultrasound frequency, an ultrasound irradiation time, an ultrasound intensity, a phase transition temperature of a material, and a mixing speed of a solution. For example, the first solution may be heated to a temperature ranging from about 25°C to about 80°C.

As an example, the first solution may be heated to a temperature ranging from about 25°C to about 80°C.

As an example, the second solution may be heated to a temperature ranging from about 50°C to about 110°C.

As an example, the first solution may be heated to a temperature ranging from about 60°C to about 80°C.

As an example, the first solution may be injected at a rate of 0.3 to 5.0 mL/min and mixed.

As an example, the second solution may be injected at a rate of 10 to 100 mL/min.

As an example, the drug delivery vehicle may have second nanobubbles formed on a surface of the shell.

As an example, the drug delivery vehicle may have a decrease in absolute zeta potential as second nanobubbles are formed on a surface of the shell.

In addition, in order to address the objects as described above, a drug delivery vehicle according to the present disclosure may be a drug delivery vehicle including a shell; and a drug and first nanobubbles contained inside the shell, the drug delivery vehicle having a uniformity within a predetermined range, wherein the uniformity of the drug delivery vehicle, represented by a poly-dispersity index (PDI), is greater than 0 and equal to or less than 0.3.

For example, a material used for the drug delivery vehicle may be one or more selected from the group consisting of lecithin, PEG-PCL (poly(ethylene glycol)-poly(ε-caprolactone)), DSPC (distearoylphosphatidylcholine), DODMA (1,2-dioleyloxy-3-(dimethylamino)propane, N,N-dimethyl-2,3-bis[(9Z)-9-octadecen-1-yloxy]-1-propanamine), PLGA (poly(lactic-co-glycolic acid)), PLA (polylactic acid), and PVA (polyvinyl alcohol).

As an example, the first nanobubbles may have an average diameter of 10 nm or more and 200 nm or less. When the average diameter of the first nanobubbles falls within the specific numerical range, the drug delivery vehicle can have excellent in vivo membrane permeability due to its small size. For example, the drug delivery vehicle may be designed to penetrate in vivo barriers with different permeabilities, such as cell membranes and the blood-brain barrier, by appropriately adjusting the average diameter of the first nanobubbles within the numerical range.

As an example, a uniformity of the drug delivery vehicle, represented by a poly-dispersity index (PDI), may be greater than 0 and equal to or less than 0.3.

As an example, a size of the drug delivery vehicle may be determined based on at least one of size control factors including a focused ultrasound frequency, an ultrasound irradiation time, an ultrasound intensity, a phase transition temperature of a material, and a mixing speed of a solution.

As an example, the drug delivery vehicle may further include second nanobubbles formed on a surface of the shell.

As an example, the drug delivery vehicle may have a decrease in absolute zeta potential as second nanobubbles are formed on a surface of the shell.

As an example, the drug delivery vehicle may use one or more materials selected from the group consisting of lecithin, cholesterol, PEG-PCL (poly(ethylene glycol)-poly(ε-caprolactone)), DSPC (distearoylphosphatidylcholine), DODMA (1,2-dioleyloxy-3-(dimethylamino)propane, N,N-dimethyl-2,3-bis[(9Z)-9-octadecen-1-yloxy]-1-propanamine), PLGA (poly(lactic-co-glycolic acid)), PLA (polylactic acid), and PVA (polyvinyl alcohol).

Hereinafter, the present disclosure will be described in more detail using the following examples. The process conditions and preparation steps not specified in the examples below may be process conditions or preparation steps that are obvious in the technical field to which the present disclosure belongs, and one skilled in the art will be able to select the foregoing without particular difficulty based on the present disclosure and reproduce the problem-solving principle of the present disclosure.

In addition, in the manufacturing method according to the present disclosure, unless otherwise specified, it should be understood that each step constituting the manufacturing method is performed at room temperature (25°C), and that each step is performed by means and tools that can be derived without particular difficulty by one skilled in the art.

### Preparation Example: Preparation of Components Used in Exemplary Embodiment of Present Disclosure

Hydrogenated soy lecithin (GL-SPC 75 H) was commercially obtained from Goshen Biotech (Namyangju, Korea) as the lecithin. A cholesterol reagent was commercially obtained from Nippon Fine Chemical (Osaka, Japan) as an auxiliary material for reinforcing the liposome structure. A 95% ethanol reagent was commercially obtained from Duksan. Distilled water was prepared with a standard of 18.2 mΩ. An ultrasonic processor FS-R01K1 from FUST Lab was used.

### Example 1: Preparation of Drug Delivery Vehicle According to Exemplary Embodiment of Present Disclosure

The lecithin (1.0 g) and the cholesterol (0.3 g) were mixed and dissolved in 70 mL of ethanol to prepare an oil-phase liposome precursor solution as the first solution. Then, the solution was mixed with 100 mL of DI water to prepare an aqueous-phase solution as the second solution. The first solution was heated to about 78°C, and the second solution was heated to about 70°C.

A high-intensity focused ultrasonic device (FS-R01K1, FUST Lab, Daejeon, Korea) was used for the focused ultrasound method. The focused ultrasonic device includes a cylindrical piezoelectric ceramic that focuses ultrasound onto a sample located at its center. Therefore, the above device enables the sample to uniformly absorb strong mechanical energy, thereby providing a more uniform dispersion force compared to other ultrasonic devices.

To maximize the ultrasonic processing effect, two lead zirconate titanate (PZT) elements were used. Both PZT frequencies were set to 380 kHz, the output of the first PZT was set to 100 W, and the output of the second PZT was set to 150 W. Using the first solution and the second solution, which were each maintained at the above temperatures, the oil-phase first solution was injected at a rate of 1.0 mL/min, and the aqueous-phase second solution was injected at a rate of 17.79 mL/min. It took about 1 hour and 40 minutes to inject all of the first solution, after which the mixed solution was circulated for additional 2 hours. The concentration of lecithin in the mixed solution was confirmed to be 5.88 g/L.

The successful preparation of liposome particles was confirmed by particle size and uniformity (PDI) analyses and cryo-EM imaging. The average size of the confirmed liposomes was 120 nm, and the uniformity value was confirmed to be 0.17 or less.

### Experimental Example 1: Visual Observation of Drug Delivery Vehicle According to Exemplary Embodiment of Present Disclosure

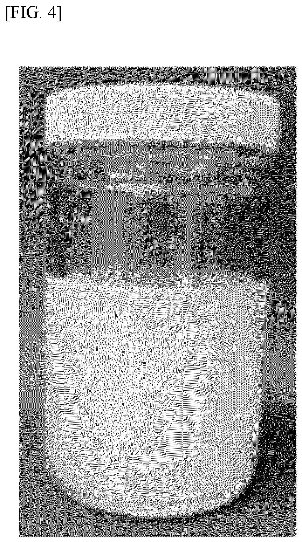

FIG. 4 is a diagram showing the results of visual observation of Example 1, a drug delivery vehicle according to an exemplary embodiment of the present disclosure. Referring to FIG. 4, it can be confirmed that the drug delivery vehicle was successfully formed in a uniform manner against a dark background. In addition, the liposome solution prepared using focused ultrasound exhibited no bubbles on the surface and showed excellent transparency. While not wishing to be bound by any particular theory, it is known that transparency correlates with the relationship between particle size and light scattering, such that larger particles scatter more light and smaller particles scatter less light.

### Experimental Example 2: Confirmation of Particle Size Distribution of Drug Delivery Vehicle According to Exemplary Embodiment of Present Disclosure

The Zetasizer Nano ZSP (Malvern Panalytical, Malvern, UK) is an analytical instrument that can measure particle size and PDI in a solution. The particle size was analyzed by measuring the scattering intensity over time in the solution under Brownian motion. The measurement was performed after diluting the liposome solution of Example 1 100-fold with DI water, and the changes in particle size and PDI of the liposomes were evaluated by performing measurements daily for four days from the date of liposome preparation. The results are shown in Table 1 below.

**[Table 1]**

| | Day 0 | Day 1 | Day 2 | Day 3 |
|---|---|---|---|---|
| Size (nm) (S.D value) | 113.6 (0.3055) | 123.8 (0.3055) | 113.7 (0.8327) | 116.5 (2.371) |
| PDI (S.D value) | 0.124 (0.011) | 0.165 (0.018) | 0.083 (0.007) | 0.060 (0.024) |

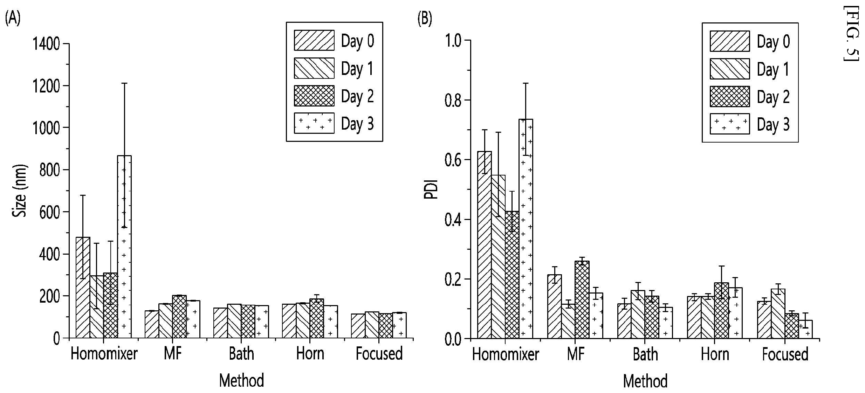

FIG. 5 shows the results of particle size and size distribution measured using DLS for Example 1, a drug delivery vehicle according to an exemplary embodiment of the present disclosure. For the liposome solution prepared using focused ultrasound (indicated as "Focused"), the liposome size measured on the day of the experiment was the smallest (113.6 nm), the solution was confirmed to be considerably stable when measured after a total of 4 days, and the PDI was measured to be about 0.1, confirming that the size distribution was very uniform. The results are shown in Table 1 below.

### Experimental Example 3: Stability Evaluation of Drug Delivery Vehicle According to Exemplary Embodiment of Present Disclosure

Turbiscan AGS (Formulaction, Toulouse, France) is a device that can analyze the stability of a solution by measuring the degree of transmission of the solution at regular time intervals to detect changes such as aggregation or phase separation. The stability of the liposome solution of Example 1 was analyzed by performing the measurements every 6 hours for one week.

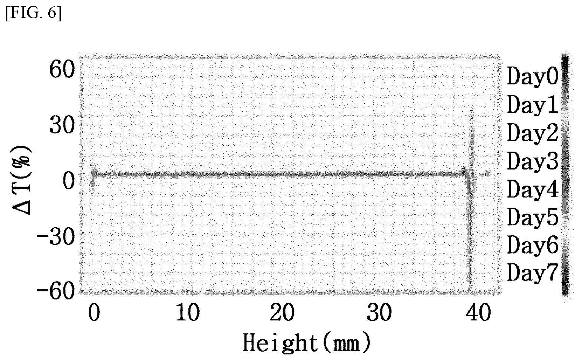

FIG. 6 is a diagram showing the results of Turbiscan, which are important results indicating the stability of the liposome solution of Example 1. FIG. 6 shows the results of measurements of the degree of delta transmission every 6 hours for one week, with the X-axis representing the height of the vial containing the sample and the Y-axis representing the change in delta transmission (%). Referring to FIG. 6, it was confirmed that the liposome solution of Example 1 exhibited a significantly low data transmission rate, indicating that the solution was highly stable with little aggregation or phase separation.

### Experimental Example 4: Cryo-EM Evaluation of Drug Delivery Vehicle According to Exemplary Embodiment of Present Disclosure

Frozen biological samples were observed by transmission electron microscopy using a Cryo-EM (Talos L 120C, FEI, Oregon, USA). Unlike other electron microscopes, Cryo-EM has the advantage of easily preventing sample deterioration, and in this experimental example, the acceleration voltage was set to 120 kV and the ice growth rate was set to less than 0.7 nm/h.

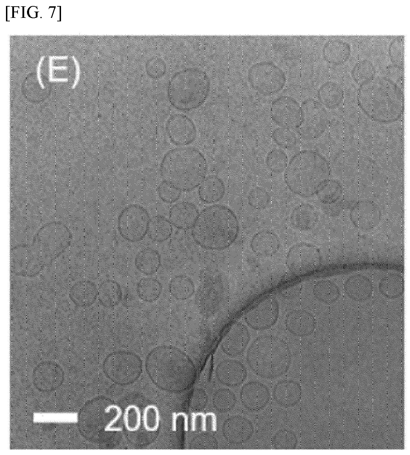

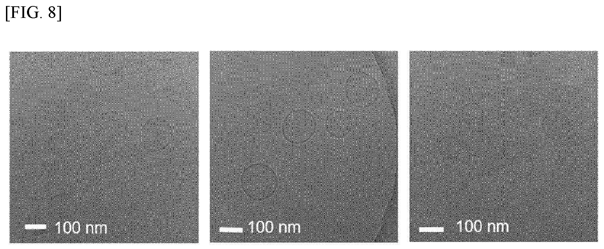

FIGS. 7 and 8 show Cryo-EM images of liposomes included in the liposome solution of Example 1. The circular shape shown in the image in FIG. 7 is a grid that appears on the Cryo-EM measurement plate. In the liposome solution of Example 1, liposomes having a size of 100 nm, which is smaller than 200 nm, were observed, and liposomes having a very uniform size distribution were confirmed to be unilamellar liposomes with uniform shape and size distribution.

Overall, it can be understood that the focused ultrasound method provides liposomes with the smallest and most uniform size, compared to similar technologies such as homogenization and high-pressure emulsification, as well as conventional ultrasonic processing methods such as bath type and horn type. This is presumed to be due to the ability of the focused ultrasound method, according to the present invention, to apply an appropriate frequency and energy to the liposome solution while delivering concentrated energy to the circulating solution. Therefore, by using the focused ultrasound method, it is possible to produce nano-sized liposomes with a uniform structure, to improve their stability, and to significantly enhance encapsulation efficiency. Such liposomes are expected to be applicable in various fields, including drug delivery and cosmetics.

While the preferred exemplary embodiments of the present invention have been described in detail, the scope of the present invention is not limited thereto, and various variations and improvements made by one skilled in the art using the basic concept of the present invention defined in the following claims also fall within the scope of the present invention.

## Claims

1. A method for manufacturing a drug delivery vehicle using focused ultrasound, the method comprising:
manufacturing a first solution;
manufacturing a second solution; and
manufacturing a third solution by mixing the first solution and the second solution and irradiating the mixture with focused ultrasound, the third solution comprising a drug delivery vehicle comprising a shell and first nanobubbles contained inside the shell.

2. The method of claim 1, wherein a uniformity of the drug delivery vehicle, represented by a poly-dispersity index (PDI), is greater than 0 and equal to or less than 0.3.

3. The method of claim 1, wherein a size of the drug delivery vehicle is determined based on at least one of size control factors comprising a focused ultrasound frequency, an ultrasound irradiation time, an ultrasound intensity, a phase transition temperature of a material, and a mixing speed of a solution.

4. The method of claim 3, wherein the first solution comprises lecithin and cholesterol.

5. The method of claim 3, wherein the first solution is heated to about 25°C to about 80°C.

6. The method of claim 5, wherein the first solution is heated to about 25°C to about 80°C.

7. The method of claim 3, wherein the second solution is heated to about 50°C to about 110°C.

8. The method of claim 7, wherein the first solution is heated to about 60°C to about 80°C.

9. The method of claim 1, wherein irradiation conditions of the focused ultrasound are 10 to 100 W and 200 to 800 kHz.

10. The method of claim 1, wherein the first solution is injected at a rate of 0.3 to 5.0 mL/min and mixed.

11. The method of claim 1, wherein the second solution is injected at a rate of 10 to 100 mL/min.

12. The method of claim 1, wherein the drug delivery vehicle has second nanobubbles formed on a surface of the shell.

13. The method of claim 4, wherein the drug delivery vehicle has a decrease in absolute zeta potential as second nanobubbles are formed on a surface of the shell.

14. A drug delivery vehicle comprising:
a shell; and
first nanobubbles contained inside the shell, the drug delivery vehicle having a uniformity within a predetermined range, wherein
the uniformity of the drug delivery vehicle, represented by a poly-dispersity index (PDI), is greater than 0 and equal to or less than 0.3.

15. The drug delivery vehicle of claim 14, wherein the drug delivery vehicle comprises one or more selected from the group consisting of lecithin, cholesterol, PEG-PCL (poly(ethylene glycol)-poly(ε-caprolactone)), DSPC (distearoylphosphatidylcholine), DODMA (1,2-dioleyloxy-3-(dimethylamino)propane, N,N-dimethyl-2,3-bis[(9Z)-9-octadecen-1-yloxy]-1-propanamine), PLGA (poly(lactic-co-glycolic acid)), PLA (polylactic acid), and PVA (polyvinyl alcohol).
